(19) Europäisches Patentamt
European Patent Office
Office européen des brevets

(11) Publication number: **0 254 632 B1**

(12) **EUROPEAN PATENT SPECIFICATION**

(45) Date of publication of patent specification: **17.06.92**

(51) Int. Cl.[5]: **C07D 405/06**, A01N 43/653, A01N 43/50, C07D 317/18, C07D 319/06, C07D 317/22

(21) Application number: **87401680.1**

(22) Date of filing: **17.07.87**

The file contains technical information submitted after the application was filed and not included in this specification

# (54) Azolyl-derivativates having fungicidal activity.

(30) Priority: **21.07.86 IT 2119486**

(43) Date of publication of application: **27.01.88 Bulletin 88/04**

(45) Publication of the grant of the patent: **17.06.92 Bulletin 92/25**

(84) Designated Contracting States: **AT BE CH DE ES FR GB LI NL SE**

(56) References cited:
**EP-A- 0 102 578**
**EP-A- 0 149 814**
**EP-A- 0 221 295**
**GB-A- 2 027 701**
**US-A- 4 338 327**

(73) Proprietor: **ISTITUTO GUIDO DONEGANI S.p.A.
Via Caduti del Lavoro
I-28100 Novara(IT)**

(72) Inventor: **Colle, Roberto
Residenza Parco 821, Milano 3
I-20089 Basiglio (MI)(IT)**
Inventor: **Ratti, Giuseppina
via Verdi 127
I-20038 Seregno (MI)(IT)**
Inventor: **Garavaglia, Carlo
piazza Vittoria 7
I-20012 Cuggiono (MI)(IT)**
Inventor: **Mirenna, Luigi
via Gamboloita4
I-20139 Milano(IT)**

(74) Representative: **Hirsch, Marc-Roger
Cabinet Hirsch 34 rue de Bassano
F-75008 Paris(FR)**

EP 0 254 632 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to azolyl-derivatives, more particularly to substituted azolyldioxanes and azolyldioxolanes, having high fungicidal activity; it also relates to the process concerning the preparation of said compounds and to the corresponding use of same in agricultural applications.

From US patent 4,338,327 are known substituted triazolyldioxolanes having general formula:

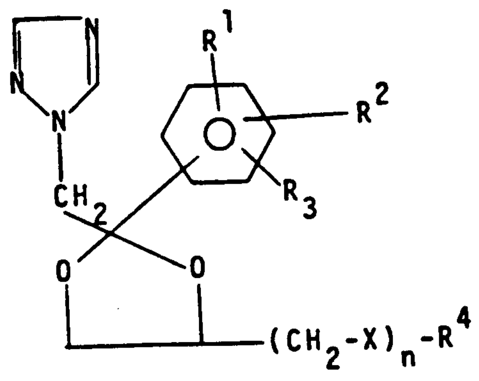

wherein:

- $R^1$, $R^2$, $R^3$ are, independently, H, an alkyl or alkoxy radical, a halogen atom, $NO_2$, CN, $CF_3$;
- n = 0 or 1;
- X = O, S;
- $R^4$ is an alkyl, mono-, di- and tri-haloalkyl, alkyloxyalkyl, mono-, di- and tri-halo-alkyloxyalkyl, lower alkenyl, 2-propynyl, 3-halo-2-propynyl, cycloalkyl, aryl, arylalkyl or arylalkenyl radical.

A class of azolyl-dioxanes and azolyl-dioxolanes has now been developed, characterized by a fluorinated substituent in the dioxanic or dioxolanic ring, and presenting a higher fungicidal activity.

Therefore, an object of the present invention consists in compounds having the general formula:

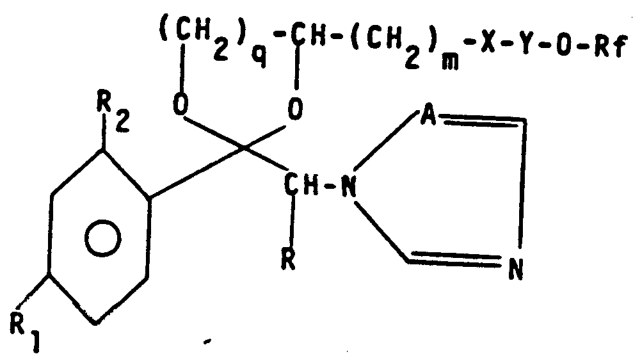

(I)

wherein:

- m = 1 or 2;
- q = 1 or 2;
- Y is a haloethylene or haloethenyl group;
- X is oxygen or sulfur;
- A = N, CH;
- R is H, $CH_3$, F;
- $R_1$ is selected from the group comprising chlorine, bromine, fluorine, $CF_3$, a phenyl, $C_1$-$C_2$ alkoxy, $C_1$-$C_2$ haloalkoxy, alkylthio, haloalkylthio radical, wherein halogen is $C_1$, Br, F;
- $R_2$ is selected from the group comprising hydrogen, chlorine, bromine, fluorine;
- $R_f$ is selected from the group comprising polyfluoroalkyl radicals having from 1 to 5 carbon atoms or polyfluoroalkenyl radicals having from 2 to 4 carbon atoms, containing at least 3 fluorine atoms and optionally other halogen atoms selected from the group comprising Cl and Br.

The compounds of the present invention contain at least 2 kyral centers.

These compounds are generally obtained in the form of diastereoisomer mixtures. These mixtures may be separated into single diastereoisomers by physical methods such as, for instance, column chromatography. Within each couple of diastereoisomers the single enantiomers may be separated by methods known in the prior art. Both diastereoisomers obtained by chromatographic separation and single enantiomers form are objects of the present invention.

The object of the present invention also includes:

- the salts of the compounds having the general formula (I) issued from an inorganic acid such as a hydrohalogenic acid, for instance hydriodic, hydrobromic, hydrochloric acid; sulfuric, nitric, thiocyanic or phosphoric acid; or from an organic acid such as acetic, propanoic, hydroxyacetic, 2-hydroxypropanoic, 2-oxopropanoic, ethanedioic, propanedioic, butanedioic, benzoic, methanesulfonic, 4-methylbenzenesulfonic acid and so on;
- the metal complexes obtained by reaction for complex formation between the derivatives of type (I) and an organic or inorganic metal salt such as halogenide, nitrate, sulfate, phosphate of, for instance, copper, manganese, zinc or iron.

Examples of compounds having the general formula (I), according to the present invention, are recorded in Table 1.

**Table 1**

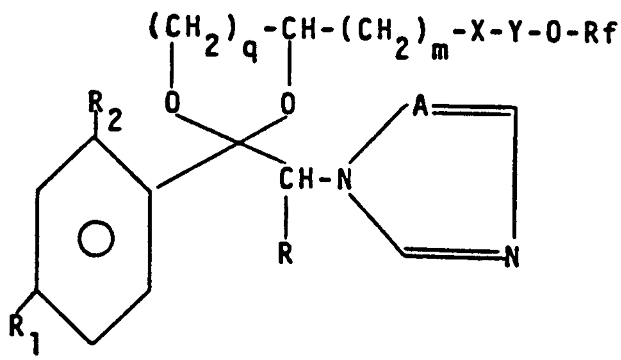

(I)

| Compound n° | R | $R_1$ | $R_2$ | q | m | X | Y | A | $R_f$ |
|---|---|---|---|---|---|---|---|---|---|
| 1 | H | Cl | Cl | 1 | 1 | O | $CF_2CFH$ | N | $CF_3$ |
| 2 | H | Cl | Cl | 1 | 2 | O | $CF_2CFH$ | N | $CF_3$ |
| 3 | H | Cl | H | 1 | 1 | O | $CF_2CFH$ | N | $CF_3$ |
| 4 | H | Cl | Cl | 1 | 1 | O | $CF_2CFH$ | N | $CF_2-CF_3$ |

The compounds having the formula (I) may be obtained according to different processes.

A process for the preparation of the compounds having the formula (I) consists in subjecting to a transketalization reaction the ketales having the formula:

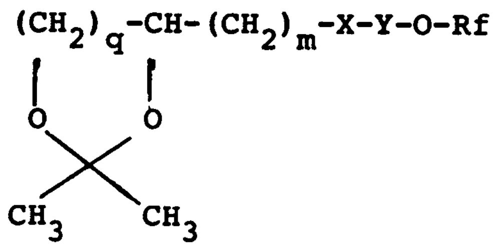

(II)

with an α-bromoketone having the formula:

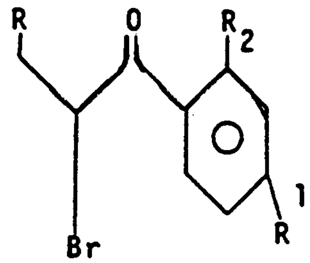

(III)

wherein R, $R^1$, $R^2$ have the above meaning;
in the presence of an acid catalyst such as sulfuric or paratoluenesulfonic acid, at a temperature ranging from 60° to 140°C and in condensing the obtained intermediate having the formula:

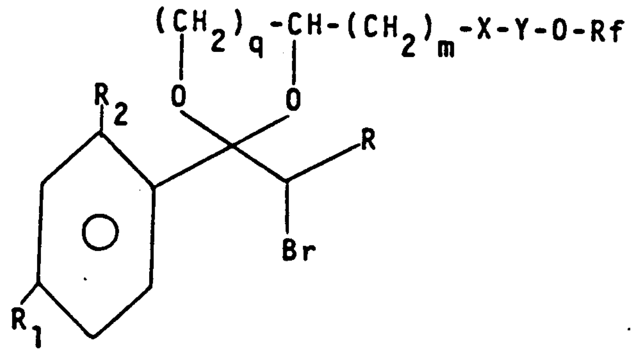

(IV)

with an alkaline salt of an azole having the formula:

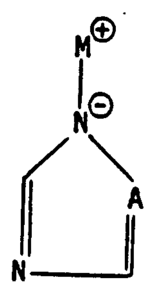

(V)

wherein:

M is an alkaline metal; and

A has the herein-before specified meaning,

in an aprotic dipolar solvent such as, for instance, dimethylformamide or dimethylsulfoxide, at a temperature comprised between 20° and the reflux temperature of the reagents.

The α-bromoketones having the formula (III) are known compounds [Lutz and al., J. Org. Chem. 12 (1947) 617; Ham, Reid, Jamieson, J.A.C.S. 52 (1930) 818; Brown, Mann, J.C.S. (1948) 847, Cowper, Davidson, Org. Synth. Coll. Vol. II (1943) 380].

Another process for preparing compounds having the formula (I) consists in subjecting to hydrolysis with aqueous mineral acids such as, for instance, HCl or $H_2SO_4$, at a temperature ranging between 20° and the boiling temperature, the ketales having the formula (II) and in allowing the thus obtained diol of formula:

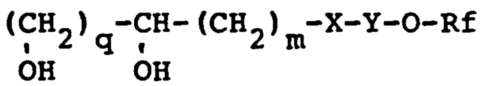

(VI)

to react with an α-azolylketone having the formula:

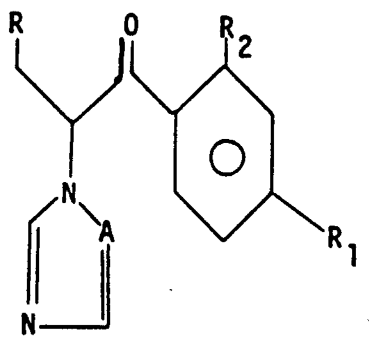

(VII)

The acetalization reaction between compounds (VI) and (VII) may be carried out according to techniques similar to those described in the prior art, for instance for preparing 2,4-diphenyl-1,3-dioxolane (Synthesis, 1974 (I) 23).

A suitable method for carrying out the reaction consists in reflux heating the reagents over many hours, by azeotropically removing the water in a suitable solvent, such as toluene or xylene, preferably in the presence of an alcohol such as butanol and in the presence of a strong acid such as 4-methylbenzenesulfonic acid. Alternatively, this reaction may be carried out, starting from simple, linear or cyclic ketales, issued from α-azolyl-ketones of type (VII), such as dimethyl or diethyl ketale or cyclopentylidene or cyclohexylydene ketale, by reaction with an excess amount of diol of type (VI), under the same experimental conditions as mentioned hereinbefore.

The intermediate compounds having the general formula (II); may be prepared according to different methods:

(a) a process for preparing the compounds having the formula (II) when Y is a haloethylene group, consists in allowing a compound of the formula:

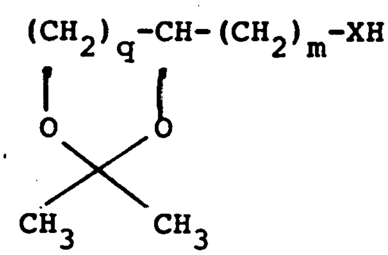

(VIII)

to react with a polyfluorinated vinyl ether having the formula:

$CX_1F = CX_2\text{-O-Rf}$ (IX)

wherein $X_1$ and $X_2$, which may be identical or different, are F, Cl, or Br and Rf has the above meaning, at a temperature comprised between 0°C and room temperature, to produce the compounds having the formula:

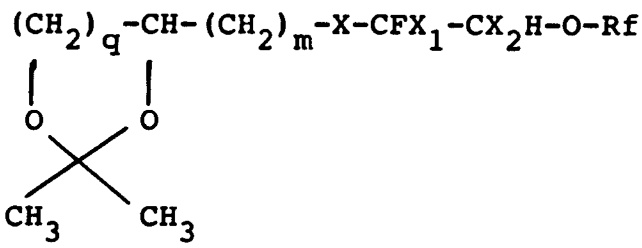

(IIa)

The compounds having the formula (VIII) may be prepared by known methods (E. Fisher, Berichte 28, 1169; A.K.M. Anisuzzan, J. Chem. Soc. (c), 1021 (1967).

The polyfluorinated vinylethers having the formula (IX) may be prepared by known methods described, for instance, in J. Org. Chem. 48, 242 (1983).

(b) A further process for preparing the intermediate compounds having the formula II, when Y is a haloethenyl group, consists in subjecting the compounds having formula (IIa) to dehydrohalogenation, to produce unsaturated compounds having the formula:

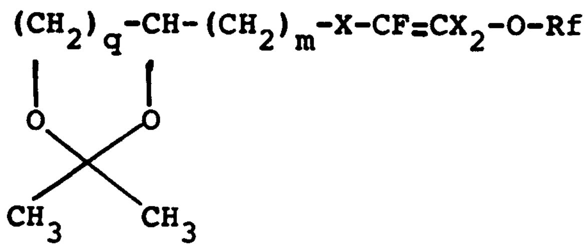

(IIb)

Moreover, the intermediates having formula (IV) may also be obtained by condensation of the compound having formula (VIII) with the α-bromoketone having formula (III) and by subsequent reaction of the product obtained having the formula:

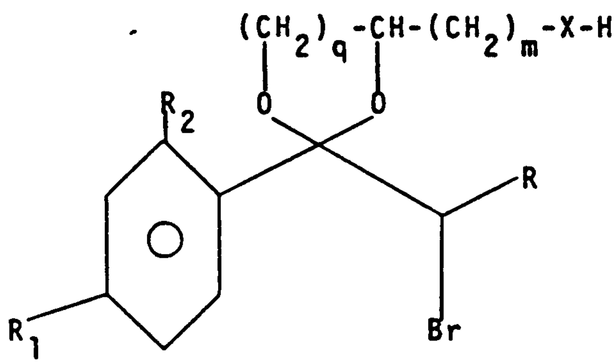

(X)

with a polyfluorinated vinylether having formula (IX), according to a reaction scheme similar to that indicated in method (a).

Finally, alternatively, the compounds having general formula (I), may be obtained by adding the compound having formula:

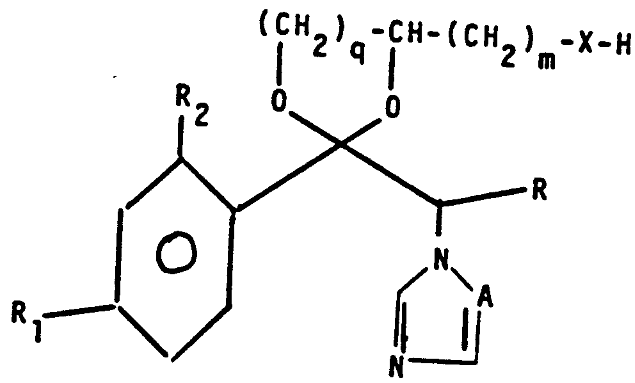

(XI)

to a polyfluorinated vinylether having formula (IX) according to a reaction scheme, similar to that indicated in method (a).

The compounds having general formula (I) are endowed with particularly high fungicidal activity against phytopathogeneous fungi, infesting the cultivation of cereals, cucurbitaceae, vine and fruit trees.

Examples of plant diseases that can be fought by the compounds of the present invention are the following:

- Erysiphe graminis on cereals,
- Sphaeroteca fuliginea on cucurbitaceae (for instance, cucumber),
- Puccinia on cereals,
- Septoria on cereals,
- Helminthosporium on cereals,
- Rhynchosporium on cereals,
- Podosphaera leucotricha on apple-trees,
- Uncinula necator on vines,
- Venturia inaequalis on apple-trees,
- Piricularia oryzae on rice,
- Botrytis cinerea,
- Fusarium on cereals,

and other diseases.

Furthermore, the compounds of formula I have other positive characteristics, such as a fungicidal activity with both a preventive and a curative character and a complete compatibility with the plants to be protected against the fungus infestation.

Besides the high fungicidal activity by preventive and curative application, the compounds having the formula (I) are characterized by systemic properties.

These properties allow the products to enter the vascular systems of the plants, and to act even in places (for instance leaves), that are very remote from those to which they are applied (for instance, roots).

For practical use in agriculture it is often useful to have a fungicidal composition available, containing one or more compounds of the formula (I) as active substance.

The application of these compositions can be made to any part of the plants, for instance on leaves, stalks, branches and roots, or on the seeds themselves, before sowing or even on the soil adjacent to the plant. Use may be made of compositions in the form of dry powders, wettable powders, emulsifiable concentrates, pastes, granulates, solutions, suspensions and so on: the choice of the composition type will depend on the specific use. The compositions are prepared according to a manner known per se, for instance by diluting or dissolving the active substance by means of a solvent medium and/or a solid diluent, optionally in the presence of surfactants. As solid diluents or carriers, use may be made of: silica, kaolin, bentonite, talc, diatomite, dolomite, calcium carbonate, magnesia, gypsum, clays, synthetic silicates, attapulgite, sepiolite. As liquid diluents, besides, of course, water, different kinds of solvents may be used, for instance aromatic solvents (benzenes, xilenes, or mixtures of alkylbenzenes), chloroaromatic solvents (chlorobenzene), paraffins (oil cuts), alcohols (methanol, propanol, butanol), amines, amides (dimethylformamide), ketones (cyclohexanone, acetophenone, isophorone, ethyl amyl ketone), esters (isobutyl acetate). As surfactants, the following may be used sodium or calcium salts or thriethanol amine of alkylsulfates, alkylsulfonates, alkylarylsulfonates, polyethoxylated alkyl-phenols, fatty alcohols condensed with ethylene oxide, polyethoxylated fatty acids, polyethoxylated sorbitol esters, polyethoxylated fats, lignin sulfonates. The compositions may also contain special additives for particular purposes, for instance

adhesive agents such as gum-arabic, polyvinyl alcohol, polyvinylpyrrolidone.

If desired, other compatible active substances, such as fungicides, phytodrugs, phytogrowth regulators, herbicides, insecticides, fertilizers, may also be added to the compositions, objects of the present invention.

The concentration of active substance in the above-mentioned compositions, may vary within a wide range, according to the active compound, cultivation, pathogen, environmental conditions and kind of formulation, that has been used. Usually the concentration of active substance ranges between 0.1 and 95, and preferably between 0.5 and 90% by weight.

The following examples will illustrate the invention.

EXAMPLE 1

Preparation of 2-(2,4-dichlorophenyl)-2-(1,2,4-triazole-1-ilmethyl)-4-(1,1,2-trifluoro-2-trifluoromethoxy-ethoxymethyl)-1,3-dioxolane (compound n° 1):

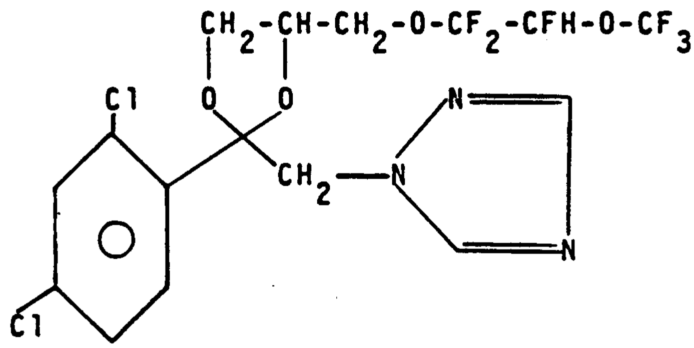

Sodium hydride (0.6 g in an oily suspension at 55-60%) was added to 2-(2,4-dichlorophenyl)-2-(1,2,4-triazole-1-ilmethyl)-4-hydroxymethyl-1,3-dioxolane (4 g) dissolved in anhydrous DMF (25 ml) and anhydrous dioxane (25 ml), at 0 °C, under nitrogen.

Then, the temperature was allowed to rise to ambient value and the whole was stirred over 30 minutes.

After having cooled the mixture to 0 °C, the apparatus was set under vacuum; trifluoro methyl-trifluoroethenyl-ether was fed thereinto and the whole was kept under an atmosphere of this gas overnight, at ambient temperature.

Then the reaction mixture was poured into water and extracted by means of methylene chloride.

The extract was rinsed with water, dried on sodium sulfate and evaporated to obtain an oil, that was analysed by silica gel chromatography, by firstly eluting with n.hexaneethyl acetate in 9:1 ratio, then in 1:1 ratio.

2.3 g of an oil were isolated, that was characterized as compound n° 1, according to the title of the present example.

Such characterization was revealed by the following spectroscopic data:

IR ($cm^{-1}$) 1510, 1280, 1210 1110.

NMR $^1H$ (60 MHz) TMS in $CCl_4$.$\delta$: 3.50-4.35 (m, 5H); 4.65 (s.broad, 2H); 6,00 (dt, 1H); 7.00-7.50 (m, 3H); 7.55 (s, 1H); 8.00 (s, 1H).

EXAMPLE 2

Preparation of 2-(2,4-dichlorophenyl)-2-(1,2,4-triazole-1-ilmethyl)-4-(1,1,2-trifluoro-2-trifluoromethoxy-ethoxyethyl)-1,3-dioxolane (compound n° 2):

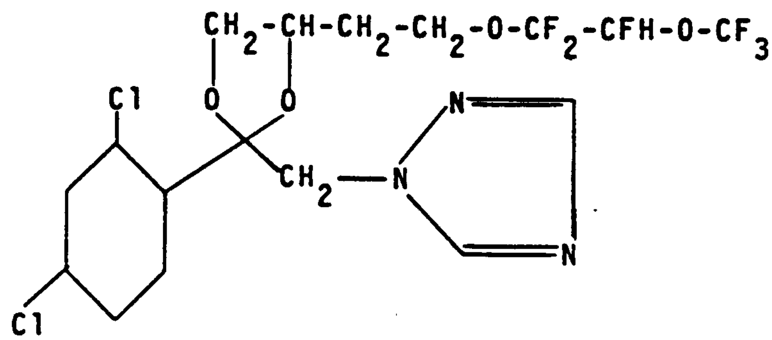

Sodium hydride (0.17 g in an oily suspension at 55-60%), was added to 2-(2,4-dichlorophenyl)-2-(1,2,4-triazole-1-ilmethyl)-4-hydroxyethyl-1,3-dioxolane (2.5 g) dissolved in anhydrous DMF (25 ml) and anhydrous THF (25 ml), under nitrogen, at 0 ° C.

Then the temperature was allowed to rise to ambient value and the whole was stirred over 15 minutes.

After having cooled the mixture to 0°, the apparatus was set under vacuum, trifluoromethyl-trifluoroethenyl-ether was fed thereinto and the whole was kept under an atmosphere of this gas overnight at ambient temperature.

Then the reaction mixture was poured into water and extracted by means of methylene chloride.

The extract was rinsed with water, dried on sodium sulfate and evaporated; the oil obtained was analysed by silica gel chromatography, by firstly eluting with 7:3 n.hexaneethyl acetate, then with ethyl acetate only.

1 g of a yellow oil was isolated, that was characterized as compound n° 2, according to the title of the present example.

Such characterization was revealed by the following spectroscopic data:

IR ($cm^{-1}$) 1490, 1270, 1210, 1100.

NMR $^1H$ (60 MHz) TMS in $CCl_4$.$\delta$: 1.40-2.00 (m, 2H); 3.05-3.50 (m, 1H); 3.70-4.40 (m, 4H); 4.65 (s.broad, 2H); 5.75 (dt, 1H); 7.10-7.50 (m, 3H); 7.65 (s, 1H); 8.00 (s, 1H).

EXAMPLE 3

Preparation of 2-(4-chlorophenyl)-2-(1,2,4-triazole-1-ilmethyl)-4-(1,1,2-trifluoro-2-trifluoromethoxy-ethoxy-methyl)-1,3-dioxolane (compound n° 3):

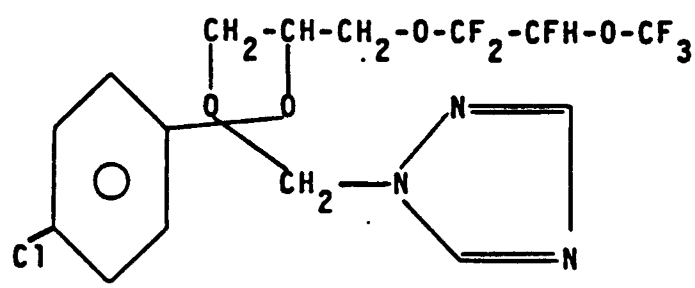

Starting from 5 g of 2-(4-chlorophenyl)-2-(1,2,4-triazole-1-ilmethyl)-4-hydroxymethyl-1,3-dioxolane, by operating as described in examples 1 and 2, 2.5 g of an oil were obtained, that was characterized as compound n° 3, according to the title of the present example.

Such characterization was revealed by the following spectroscopic data.

NMR $^1H$ (60 MHz) TMS in $CCl_4$.$\delta$: 3.25-4.10 (m, 5H); 4.90 (s.broad, 2H); 5.55 (dm, 1H); 7.10-7.50 (m, 4H); 7.80 (s, 1H); 8.05 (s, 1H).

EXAMPLE 4

Preparation of 2-(2,4-dichlorophenyl)-2-(1,2,4-triazole-1-ilmethyl)-4-(1,1,2-trifluoro-2-perfluoroethoxy-ethoxy-methyl)-1,3-dioxolane (compound n° 4).

Starting from 3 g of 2-(2,4-dichlorophényl)-2-(1,2,4-triazole-1-ilmethyl)-4-hydroxymethyl)-1,3-dioxolane in atmosphere of $C_2F_5O\text{-}CF=CF_2$, by operating as described in the preceding examples, 2.3 g of an oil were obtained, that was characterized as compound n° 4, according to the title of the present example.

Such characterization was revealed by the following spectroscopic data.

IR ($cm^{-1}$): 1510, 1280, 1230, 1100.

NMR $^1H$ (60 MHz) TMS in $CCl_4$.$\delta$: 3.5-4.30 (m, 5H); 4.75 (s.broad, 2H); 5.65 (dt, 1H); 7.10-7.60 (m, 3H); 7.85 (s, 1H); 8.10 (s, 1H).

EXAMPLE 5

Determination of the fungicidal activity against Cucumber oidium (Sphaerotheca fuliginea "Schlech" Salmon)

Preventive activity:

Cucumber plants cv. Marketer, grown in pots in a conditioned environment, were sprinkled on the lower leaf faces with the products being tested in a water-acetone solution containing 20% of acetone (vol./vol.). Then, the plants were kept in a conditioned environment over 1 day and thereafter sprinkled on the upper leaf faces with an aqueous suspension of conidia of Sphaerotheca fuliginea (200.000 conidia/ml). The plants were then carried back into a conditioned environment.

At the end of the incubation period of the fungus (8 days), the infection degree was evaluated according to indice of a valuation scale ranging from 100 (= sound plant) to 0 (= wholly infected plant).

Curative activity:

Cucumber plants cv. Marketer, grown in pots in a conditioned environment, were sprinkled on the upper leaf faces with an aqueous suspension of conidia of Sphaerotheca fuliginea (200.000 conidia/ml). 24 hours after the infection the plants were treated with the products being tested in a water-acetone solution containing 20% of acetone (vol./vol.) by sprinkling both leaf faces.

At the end of the incubation period of the fungus (8 days), during which time the plants were kept in a suitably conditioned environment, the infection degree was evaluated according to a valuation scale ranging from 100 (= sound plan) to 0 (wholly infected plant).

The results are recorded in Table 2.

EXAMPLE 6

Determination of the fungicidal activity against wheat oidium (Erysiphe Graminis D.C.).

Preventive activity:

Leaves of wheat cv. Irnerio, grown in pots in a conditioned environment, were sprayed on both leaf faces with the products being tested in a water-acetone solution containing 20% of acetone (vol./vol.).

After maintaining 1 day in a conditioned environment at 20°C and 70% of relative humidity, the plants were sprayed on both leaf faces with an aqueous suspension of Erysiphe Graminis (200.000 conidia/cc). After maintaining 24 hours in an moisture-saturated environment at 21°C, the plants were kept in a conditioned environment for the fungus incubation.

At the end of this period (12 days) the infection degree was evaluated at sight, according to a scale ranging from 100 (sound plant) to 0 (wholly infected plant).

Curative activity:

Leaves of wheat cv. Irnerio, grown in pots in a conditioned environment, were sprayed on both leaf faces with an aqueous suspension of Erysiphe Graminis (200.000 conidia/cc). After a stay time of 24 hours in an environment saturated with moisture, at 21°C, the leaves were treated with the products being tested in a water-acteone solution containing 20% of acetone (vol./vol.) by spraying both leaf faces.

At the end of the incubation period (12 days), the infection degree was evaluated at sight according to a valuation scale ranging from 100 (= sound plant) to 0 (= wholly infected plant).

The results are recorded in Table 2.

TABLE 2

| Compound No | Dose g/l | *Sphaeroteca fuliginea/cucumber* Preventive activity | *Sphaeroteca fuliginea/cucumber* Curative activity | Erysiphe graminis trit. /wheat Preventive activity | Erysiphe graminis trit. /wheat Curative activity |
|---|---|---|---|---|---|
| 1 | 0.5 | 100 | 100 | 100 | 100 |
| | 0.25 | 100 | 100 | 100 | 100 |
| | 0.125 | 100 | 100 | 100 | 100 |
| | 0.06 | 100 | 100 | 100 | 100 |
| 2 | 0.5 | 100 | 100 | 100 | 100 |
| | 0.25 | 100 | 100 | 100 | 100 |
| | 0.125 | 100 | 100 | 100 | 100 |
| | 0.06 | 100 | 100 | 100 | 100 |

**Claims**

**1.** Compounds having the general formula:

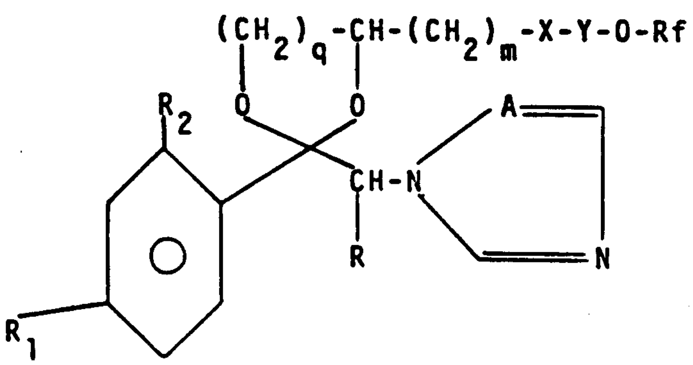

(I)

wherein:

- m = 1 or 2:
- q = 1 or 2:
- Y is a haloethylene or haloethenyl group;
- X is oxygen or sulfur;
- A = N, CH;
- R is H, $CH_3$, F;
- $R_1$ is selected from the group comprising chlorine, bromine, fluorine, $CF_3$, a phenyl, $C_1$-$C_2$ alkoxy, $C_1$-$C_2$ haloalkoxy, alkylthio, haloalkylthio radical, wherein halogen is C1, Br, F;

$R_2$ is selected from the group comprising: H, chlorine, bromine, fluorine;

$R_f$ is a $C_1$-$C_5$ polyfluoroalkyl or $C_2$-$C_4$ polyfluoroalkenyl radical containing at least 3 fluorine atoms and optionally other halogen atoms selected from the group comprising Cl and Br.

**2.** Compound according to claim 1, i.e. 1-[2-(2,4-dichlorophenyl)-4-(1,1,2-trifluoro-2-trifluoromethoxyethoxymethyl)1,3-dioxolane-2-il-methyl]-1H-1,2,4-triazole.

**3.** A process for preparing compounds having the formula (I), according to claim 1, consisting in subjecting to transketalization reaction the ketales having the formula:

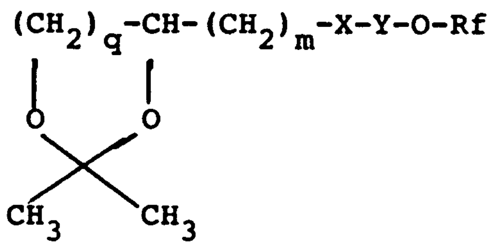

(II)

with an α-bromo ketone having the formula:

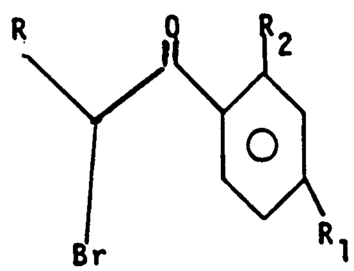

(III)

wherein R, $R^1$, $R^2$ have the meaning specified in claim 1, in the presence of an acid catalyst, at a temperature ranging from 60° to 140°C and in condensing the obtained intermediate having the formula:

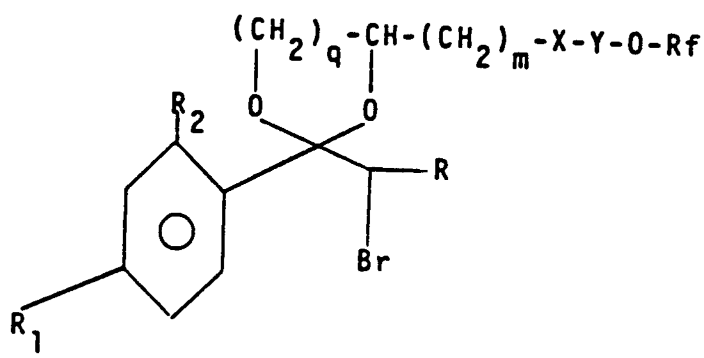

(IV)

with an alkaline salt of an azole having the formula:

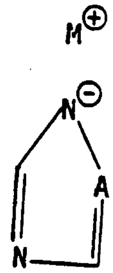

(V)

wherein:

M is an alkaline metal; and

A has the herein-before specified meaning,

in an aprotic dipolar solvent, at a temperature ranging from 20 ° C to the reflux temperature of the reagents.

**4.** A process for preparing the compounds having the formula (I), according to claim 1, consisting subjecting to hydrolysis by means of aqueous mineral acids, at a temperature ranging from 20 ° C to boiling temperature, the ketales having the formula (II), according to claim 3, and in allowing the diol thus obtained of formula:

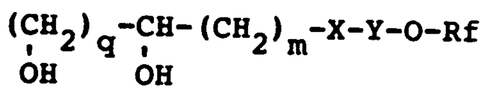

(VI)

to react with an α-azolylketone having the formula:

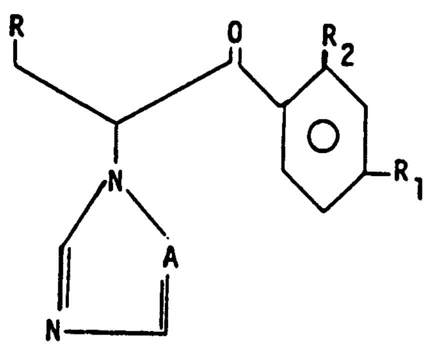

(VII)

in an organic solvent, optionally in the presence of an aliphatic alcohol, in the presence of a strong acid, at the reflux temperature of the mixture, by azeotropically removing the water formed during the reaction.

**5.** Compounds having the formula:

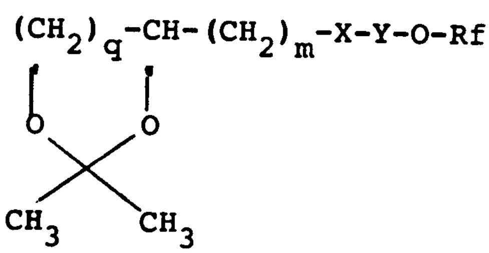

(II)

wherein m, n, q, X, Y and Rf have the meanings specified in claim 1.

6. A process for preparing the compounds having the formula (II), according to claim 5, when Y is a haloethylene group, consisting in allowing a compound of formula:

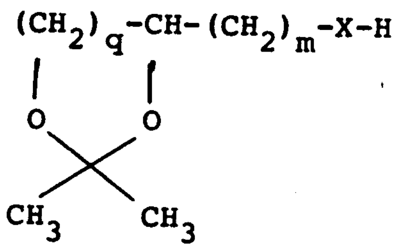

(VIII)

wherein m, q and X have the meanings specified in claim 1, react with a polyfluorinated vinylether having the formula:

$CX_1F = CX_2\text{-O-RF}$ (IX)

wherein:

$X_1$ and $X_2$, which may be identical or different, are F, Cl or Br; and

Rf has the meaning specified in claim 1,

at a temperature ranging from 0 ° C to ambient temperature, to produce compounds having the formula:

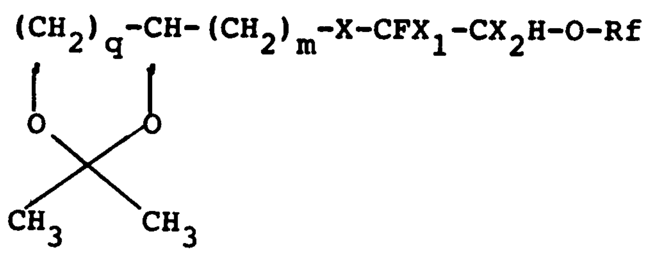

(IIa)

7. A process for preparing compounds having the formula (II), according to claim 5, when Y is a haloethenyl group, consisting in subjecting to dehydrohalogenation the compounds having the formula (IIa), according to claim 6, to produce unsaturated compounds having the formula:

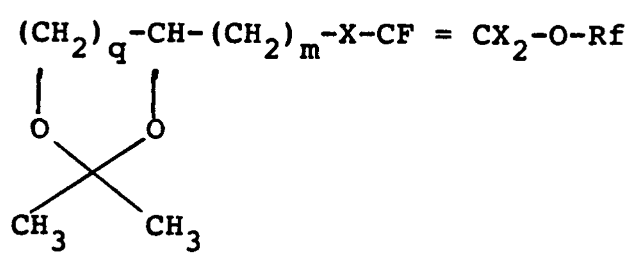

(IIb)

8. A method of fighting fungus infections in useful plants consisting in distributing on the plant, on the seeds or on the surrounding soil, when fungus infestation is foreseen or is already in progress, an effective amount of a compound, according to claim 1, either as such or in the form of a suitable composition.

9. A method of fighting fungus infections in useful plants consisting in distributing on the plant, on the seeds or on the surrounding soil, when fungus infection is foreseen or is already in progress, an effective amount of 1-[2-(2,4-dichlorophenyl)-4-(1,1,2-trifluoro-2-trifluoro-methoxy-ethoxymethyl)-1,3-dioxolane-2-ilmethyl]-1H-1,2,4-triazole compound, according to claim 2, either as such or in the form of a suitable composition.

**10.** Fungicidal compositions having as active ingredient one or more compounds, according to claim 1, together with a solid or liquid carrier and, optionally other additives.

**11.** Fungicidal compositions having, as active ingredient, 1-[2-(2,4-dichlorophenyl)-4-(1,1,2-trifluoro-2-trifluoromethoxyethoxymethyl)-1,3-dioxolane-2-ilmethyl]-1H-1,2,4-triazole compound, together with a solid or liquid carrier and, optionally, other additives.

**Revendications**

**1.** Dérivés répondant à la formule générale:

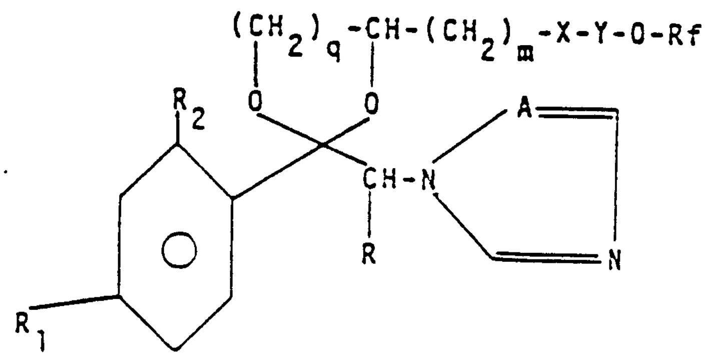

(I)

dans laquelle:

- m = 1 ou 2;
- q = 1 ou 2;
- y représente un groupe haloéthylène ou haloéthényle;
- x représente un atome d'oxygène ou de soufre;
- A = N, CH;
- R représente H, $CH_3$, F;
- $R_1$ est choisi dans le groupe formé par les atomes de chlore, de brome, de fluor, $CF_3$, les radicaux phényle, alcoxy en $C_1$ à $C_2$, haloalcoxy en $C_1$ à $C_2$, alkylthio, haloalkylthio, l'halogène étant Cl, Br, F;
- $R_2$ est choisi dans le groupe formé par H, un atome de chlore, de fluor, de brome;
- $R_f$ représente un radical polyfluoroalkyle en $C_1$ à $C_5$ ou polyfluoroalkényle en $C_2$ à $C_4$ renfermant au moins 3 atomes de fluor et éventuellement un autre atome d'halogène choisi dans le groupe formé par Cl et Br.

**2.** Dérivé selon la revendication 1, c'est-à-dire 1-[2-(2,4-dichlorophényl)-4-(1,1,2-trifluro-2-trifluorométhoxyéthoxyméthyl)-1,3-dioxolane-2-il-méthyl]-1H-1,2,4-triazole.

**3.** Un procédé de préparation de dérivés répondant à la formule (I) selon la revendication 1, consistant à soumettre à une réaction de transcétalisation les cétals répondant à la formule:

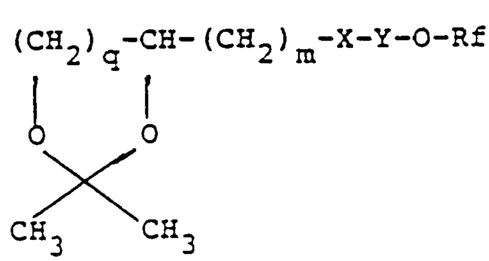

(II)

avec une α-bromocétone répondant à la formule:

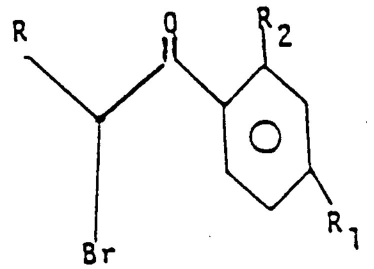

(III)

dans laquelle R, $R^1$, $R^2$ présentent les significations spécifiées dans la revendication 1, en présence d'un catalyseur acide, à une température comprise entre 60 et 140 ° C et à condenser l'intermédiaire obtenu répondant à la formule:

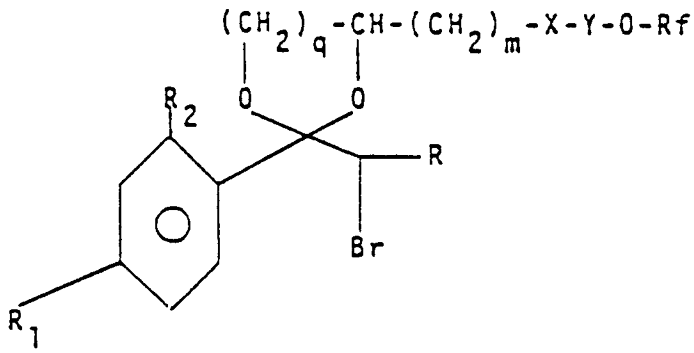

(IV)

avec un sel alcalin d'un azole répondant à la formule:

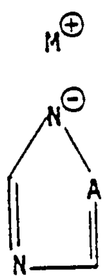

(V)

dans laquelle:

M représente un métal alcalin; et

A présente la signification spécifiée ci-dessus,

dans un solvant dipolaire aprotique, à une température comprise entre 20 ° C et la température de reflux des réactifs.

**4.** Un procédé de préparation des dérivés répondant à la formule (I), selon la revendication 1, consistant à soumettre à une hydrolyse au moyen d'acides minéraux aqueux, à une température comprise entre 20 ° C et la température d'ébullition, les cétals répondant à la formule (II) selon la revendication 3, et en faisant réagir le diol ainsi obtenu de formule:

$(CH_2)_q-CH-(CH_2)_m-X-Y-O-Rf$
$\quad | \qquad\quad |$
$\;\; OH \quad\;\; OH$

(VI)

avec une α-azolylcétone répondant à la formule:

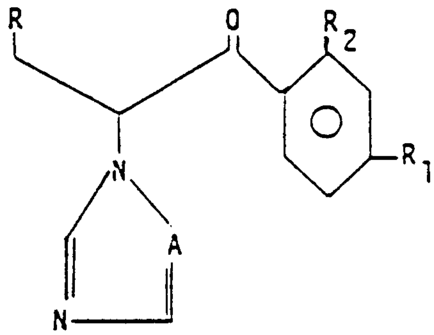

(VII)

dans un solvant organique, éventuellement en présence d'un alcool aliphatique, en présence d'un acide fort, à la température de reflux du mélange, en éliminant par distillation azéotropique l'eau formée pendant la réaction.

**5.** Dérivés répondant à la formule (II):

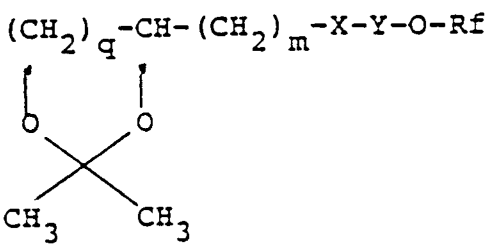

(II)

dans laquelle m, n, q, X, Y et Rf présentent les significations spécifiées dans la revendication 1.

**6.** Un procédé de préparation de dérivés répondant à la formule (II) selon la revendication 5, dans laquelle Y représente un groupe haloéthylène, consistant à faire réagir un dérivé de formule:

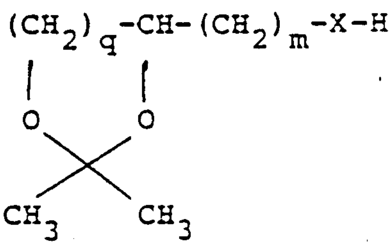

(VIII)

dans laquelle m, q et X présentent les significations indiquées dans la revendication 1, avec un vinyléther polyfluoré répondant à la formule:

$CX_1F = CX_2\text{-O-Rf}$ (IX)

dans laquelle:
$X_1$ et $X_2$ qui peuvent être identiques ou différents représentent F, Cl ou Br; et
Rf présente la signification indiquée dans la revendication 1,
à une température comprise entre 0 ° C et la température ambiante, pour obtenir les dérivés répondant à la formule;

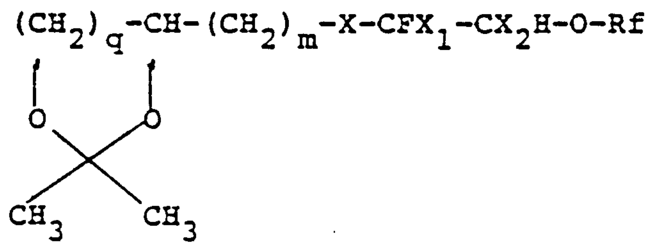

(IIa)

7. Un procédé de préparation de dérivés répondant à la formule (II) selon la revendication 5, dans laquelle Y représente un groupe haloéthényl, consistant à soumettre à une déshydrohalogénation les dérivés répondant à la formule (IIa), selon la revendication 6, pour obtenir des dérivés insaturés répondant à la formule

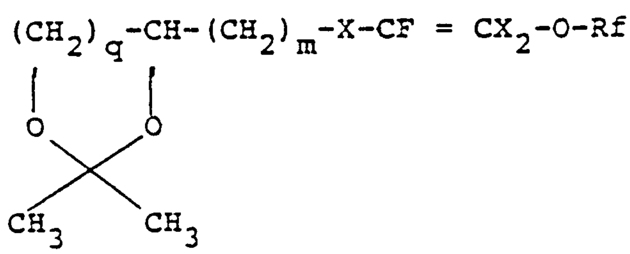

(IIb)

8. Un procédé de lutte contre les infections fongiques de plantes utiles consistant à distribuer sur les plantes, sur les graines ou sur le sol environnant, lorsque l'infestation fongique est prévue ou qu'elle est déjà en cours, une quantité efficace d'un dérivé selon la revendication 1, soit en tant que tel, soit sous la forme d'une composition convenable.

9. Un procédé de lutte contre les infections par échantillons de plants utiles constituant à distribuer sur les plantes, sur les graines ou sur le sol environnant, lorsque l'infection est prévue ou déjà en cours, une quantité efficace de 1-[2-(2,4-dichlorophényl)-4-(1,1,2-trifluro-2-trifluoro-méthoxy-éthoxyméthyl)-1,3-dioxolane-2-il-méthyl]-1H-1,2,4-triazole, selon la revendication 2, soit en tant que tel, soit sous la forme d'une composition convenable.

10. Compositions fongiques renfermant comme ingrédients actifs un ou plusieurs dérivés selon la revendication 1, en même temps qu'un support solide ou liquide et éventuellement d'autres additifs.

11. Compositions fongiques renfermant comme ingrédients actifs le 1-[2-(2,4-dichlorophényl)-4-(1,1,2-trifluro-2-trifluoro-méthoxy-éthoxyméthyl)-1,3-dioxolane-2-il-méthyl]-1H-1,2,4-triazole en même temps qu'un support solide ou liquide et éventuellement d'autres additifs.

## Patentansprüche

1. Verbindungen mit folgender allgemeinen Formel:

(I)

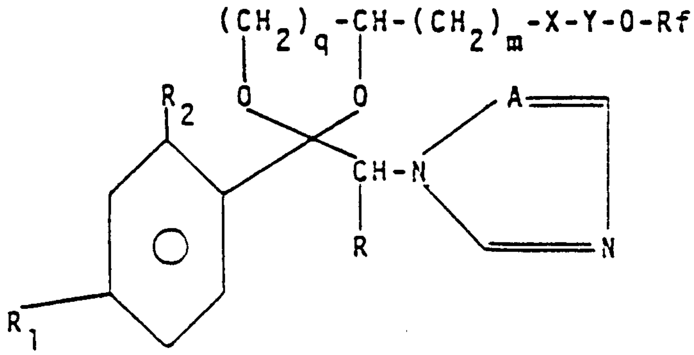

in welchen:

- m = 1 oder 2;
- q = 1 oder 2;
- Y = eine Haloäthylen- oder Haloäthenylgruppe;
- X = Sauerstoff oder Schwefel;
- A = N, CH;
- R = H, $CH_3$, F;
- $R_1$ eine der folgenden Gruppen: Chlor, Brom, Fluor, $CF_3$, Phenyl-, $C_1$-$C_2$-alkoxy-, Halo-($C_1$-$C_2$-alkoxy)-, Alkylthio-, Haloalkylthioradikal, wobei das Halogen Cl, Br oder F ist;
- $R_2$ eine der folgenden Gruppen: H, Chlor, Brom und Fluor ist,
- $R_f$ ein Polyfluoro- ($C_1$-$C_5$-alkyl)- oder ein Polyfluoro($C_2$-$C_4$-alkenyl)Radikal das wenigstens 3 F-Atome, sowie ggf. Cl- oder Br-Atome enthält.

**2.** Verbindung nach Anspruch 1, nämlich 1-[2-(2,4-Dichlorophenyl)-4-(1,1,2-trifluoro-2-trifluoromethoxyäthoxymethyl)-1,3-dioxolan-2-il-methyl]-1H-1,2,4-triazol.

**3.** Ein Verfahren zur Herstellung von Verbindungen mit der Formel (I) nach Anspruch 1, bei dem man Ketale der Formel:

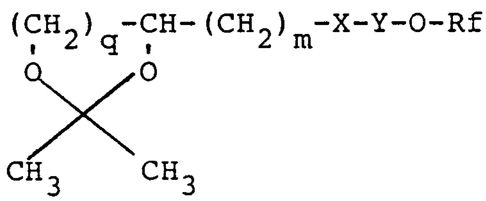

(II)

einer Transketalisations-Reaktion mit einem α-Bromoketon der Formel:

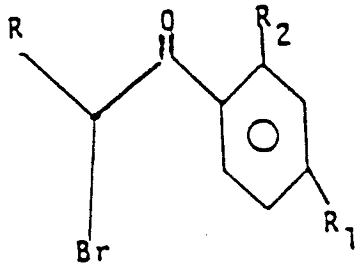

(III)

unterwirft, in welcher Formel (II) R, $R^1$ und $R^2$ die gleiche Bedeutung haben wie in Anspruch 1, bei Anwesenheit eines saueren Katalysators bei einer Temperatur von 60° bis 140°C und wobei man das anfallende Zwischenprodukt der Formel:

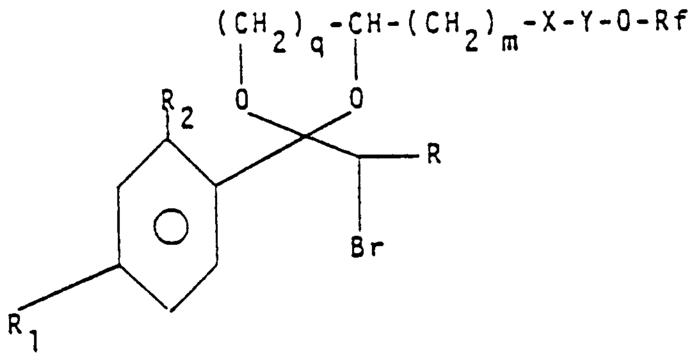

(IV)

einer Kondensation unterwirft mit einem Alkalisalz eines Azols der Formel:

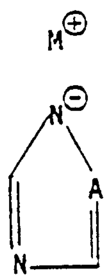

(V)

in welcher:

M ein Alkalinmetall; und

A die vorstehend angezeigte Bedeutung hat,

und wobei diese Kondensation in einem aprotischen dipolaren Lösungsmittel bei einer zwischen 20°C und der Rückflusstemperatur der Reagenzien liegenden Temperatur durchgeführt wird.

**4.** Ein Verfahren zur Herstellung von Verbindungen der Formel (I) nach Anspruch 1, bei welchem man die Ketale gemäss der Formel (II) in Anspruch 3 vermittels anorganischer wässeriger Säuren bei einer zwischen 20°C und dem Siedepunkt liegenden Temperatur einer Hydrolyse unterwirft und sodann das hierbei anfallende Diol der Formel:

$$\underset{OH}{(CH_2)_q}-\underset{OH}{CH}-(CH_2)_m-X-Y-O-Rf \qquad (VI)$$

mit einem $\alpha$-Azolylketon der Formel:

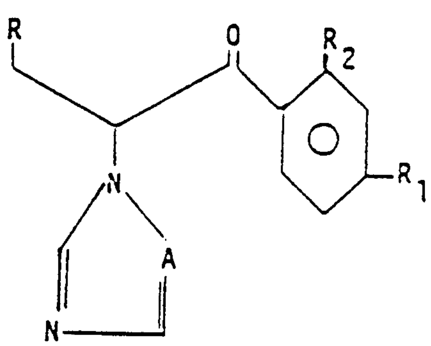

(VII)

reagieren lässt in einem organischen Lösungsmittel, in Gegenwart einer starken Säure und ggf. in Gegenwart eines aliphatischen Alkohols, bei der Rückflusstemperatur des Gemischs, wobei das während der Reaktion gebildete Wasser azeotropisch entfernt wird.

**5.** Verbindungen gemäss der Formel:

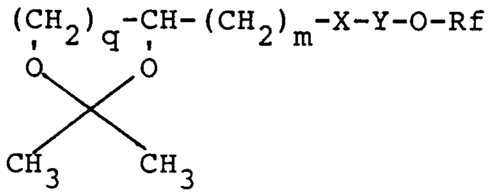

(II)

in welcher m, n, q, x, y, und Rf die in Anspruch 1 definierte Bedeutung haben.

**6.** Verfahren zur Herstellung der Verbindungen gemäss der Formel (II) nach Anspruch 5, in welcher Y eine Haloäthylengruppe darstellt, bei dem man eine Verbindung der Formel:

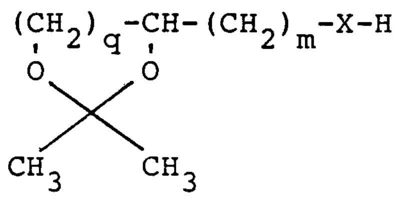

(VIII)

in welcher m, q und X die in Anspruch 1 definierte Bedeutung haben, bei einer zwischen 0 °C und Raumtemperatur liegenden Temperatur reagieren lässt mit einem mit Polyfluor angereicherten Vinyläther der Formel:

$CX_1F = CX_2\text{-O-Rf}$ (IX)

wo:
$X_1$ und $X_2$ identisch oder verschieden, F, Cl oder Br darstellen; und
Rf die in Anspruch 1 angegebene Bedeutung hat, um Verbindungen der Formel:

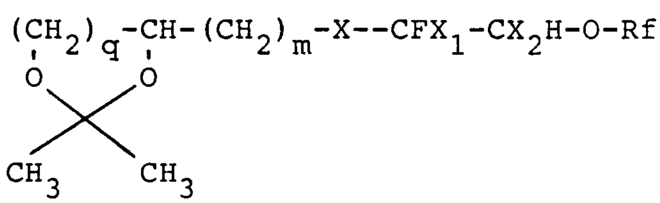

(IIa)

zu erhalten.

**7.** Ein Verfahren zur Herstellung von Verbindungen gemäss der Formel (II) nach Anspruch 5, in welcher Y eine Haloäthylengruppe darstellt, bei dem die Verbindungen gemäss der Formel (IIa) nach Anspruch 6 deshydrohalogeniert um ungesättigte Verbindungen mit der Formel:

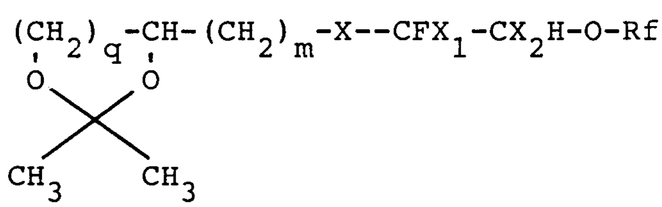

(IIb)

zu erhalten.

**8.** Ein Verfahren zu Bekämpfung des Fungusbefalls von Nutzpflanzen, bei dem man, wenn der Fungusbefall vorausgesehen oder bereits festgestellt wird, eine wirksame Menge einer Verbindung nach Anspruch 1 in Form eines Reinprodukts oder einer geeigneten Zusammensetzung auf die Pflanze, auf die Saat oder auf den umgebenden Erdboden aufbringt.

**9.** Ein Verfahren zu Bekämpfung des Fungusbefalls von Nutzpflanzen, bei dem man, wenn der Fungusbefall vorausgesehen oder bereits festgestellt wird, eine wirksame Menge von 1-[2-(2,4-Dichlorophenyl)-4-(1,1,2-trifluoro-2-trifluoro-methoxy äthoxy-methyl)-1,3-dioxolan-2-il-methyl]-1H-1,2,4-triazol nach Anspruch 2 entweder Form eines Reinprodukts oder in Form einer geeigneten Zusammensetzung auf die Pflanze, auf die Saat oder auf den umgebenden Erdboden aufbringt.

**10.** Fungusbekämpfungszusammensetzungen mit einem Wirkstoff in Form einer oder mehrerer Verbindungen nach Anspruch 1, kombiniert mit einem festen oder flüssigen Trägerstoff und ggf. mit anderen Zusatzstoffen.

**11.** Fungusbekämpfungszusammensetzungen, die als Wirkstoff 1-[2-(2,4-Dichlorophenyl)-4-(1,1,2-trifluoro-2-trifluoromethoxyäthoxymethyl)-1,3-dioxolan-2-il-methyl]-1H-1,2,4-triazol in Kombination mit einem festen oder flüssigen Trägerstoff und ggf. mit anderen Zusatzstoffen enthalten.